(19) Europäisches Patentamt / European Patent Office / Office européen des brevets

(11) **EP 3 214 064 B1**

(12) **EUROPEAN PATENT SPECIFICATION**

(45) Date of publication and mention
of the grant of the patent:
**09.10.2019 Bulletin 2019/41**

(51) Int Cl.:
**C07C 51/60** (2006.01)     **C07C 51/64** (2006.01)
**C07C 61/09** (2006.01)

(21) Application number: **16182815.7**

(22) Date of filing: **04.08.2016**

(54) **METHOD FOR PREPARING 1,4-CYCLOHEXANEDICARBOXYLIC ACID DICHLORIDE**

VERFAHREN ZUR HERSTELLUNG VON 1,4-CYCLOHEXANEDICARBOXYL-PHTHALSÄUREDICHLORID

MÉTHODE DE PRÉPARATION DU DICHLORURE D'ACIDE 1,4-CYCLOHEXANEDICARBOXYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.03.2016 JP 2016041804**

(43) Date of publication of application:
**06.09.2017 Bulletin 2017/36**

(73) Proprietor: **Toray Fine Chemicals Co., Ltd.**
**Tokyo 101-0041 (JP)**

(72) Inventors:
• **Sugawara, Kazuki**
 **Tokai-shi, Aichi 476-8567 (JP)**
• **Nishikawa, Takeshi**
 **Tokai-shi, Aichi 476-8567 (JP)**

(74) Representative: **Kador & Partner PartG mbB**
**Corneliusstraße 15**
**80469 München (DE)**

(56) References cited:
**JP-A- 2014 047 201     JP-A- 2014 185 119**
**JP-A- 2014 196 288     JP-A- 2015 172 036**
**US-A- 3 631 107**

**Description**

Technical Field

The present invention relates to methods for preparing 1,4-cyclohexanedicarboxylic acid dichloride. Particularly, the invention relates to a preparation method that preferentially provides *cis* isomers, having a low melting point and industrially easy to handle, inhibits isomerization from *cis* to *trans* during the preparation, and efficiently removes acidic impurities contained.

Background Art

1,4-Cyclohexanedicarboxylic acid dichloride is an important compound used as a monomer for synthetic resins, a raw material of resin additives, and a raw material of pharmaceutical intermediates.

1,4-Cyclohexanedicarboxylic acid dichloride includes *cis* and *trans* isomers. The *cis* isomer has a lower melting point than the *trans* isomer. 1,4-Cyclohexanedicarboxylic acid dichloride predominantly containing *cis* isomers is liquid at normal temperature and easy to use as an industrial raw material, whereas 1,4-cyclohexanedicarboxylic acid dichloride predominantly containing *trans* isomers is solid at normal temperature and, when used as an industrial raw material, needs to be dissolved by heating, which is a troublesome operation. Thus, there is a need to prepare 1,4-cyclohexanedicarboxylic acid dichloride predominantly containing *cis* isomers and liquid at normal temperature, which can be used as a general-purpose industrial raw material.

In general, 1,4-cyclohexanedicarboxylic acid dichloride is prepared by converting 1,4-cyclohexanedicarboxylic acid into an acid chloride using a chlorinating agent, such as phosgene, oxalyl chloride, phosphorus trichloride, phosphorus pentachloride, or thionyl chloride, and purifying the acid chloride by distillation.

Typically, 1,4-cyclohexanedicarboxylic acid, used as a reactant, is prepared by nuclear hydrogenation of terephthalic acid (see, for example, Patent Document 1). The 1,4-cyclohexanedicarboxylic acid prepared by this method is a *cis/trans* mixture composed mainly of *cis* isomers. When such 1,4-cyclohexanedicarboxylic acid predominantly containing *cis* isomers is converted into 1,4-cyclohexanedicarboxylic acid dichloride using a chlorinating agent, heat is applied to promote the reaction and during distillation operation. Due to the heat, *cis*-1,4-cyclohexanedicarboxylic acid dichloride isomerizes into *trans*-1,4-cyclohexanedicarboxylic acid dichloride. For example, a method is known for preparing 1,4-cyclohexanedicarboxylic acid dichloride predominantly containing *trans* isomers by adding thionyl chloride to 1,4-cyclohexanedicarboxylic acid containing *cis* and *trans* isomers and heating the resulting mixture in the presence of a strong acid (Patent Document 2).

There is disclosed a method for preparing 1,4-cyclohexanedicarboxylic acid dichloride predominantly containing *cis* isomers by performing the reaction at 80°C or lower to inhibit isomerization from *cis* to *trans* and filtering off residual terephthalic acid impurities (Patent Document 3). In this preparation method, however, impurities derived from phthalic acid must be filtered off from the 1,4-cyclohexanedicarboxylic acid dichloride obtained by the reaction. In the presence of water, 1,4-cyclohexanedicarboxylic acid dichloride is unstable and decomposed to generate hydrochloric acid, and therefore is very difficult to industrially prepare. Furthermore, 1,4-cyclohexanedicarboxylic acid dichloride, when chlorinated using, for example, thionyl chloride, is colored yellow during the reaction. The filtering alone can remove impurities but not the yellow coloration, and thus cannot provide a colorless 1,4-cyclohexanedicarboxylic acid dichloride.

When a chlorinating agent such as thionyl chloride is used in the reaction, acidic gas, such as sulfur dioxide, generated during the reaction is contained in the reaction system. Such acidic gas is preferably reduced as much as possible because it has an adverse effect in a subsequent reaction using 1,4-cyclohexanedicarboxylic acid dichloride. Typically, the acidic gas contained can be removed by thermal degassing under normal reduced pressure. Since 1,4-cyclohexanedicarboxylic acid dichloride predominantly containing *cis* isomers is a viscous liquid, acidic gas generated by the reaction is contained in the viscous liquid, and to remove the acidic gas, it is necessary to raise the temperature. As the temperature is raised, however, *cis* isomers transform into *trans* isomers, leading to a reduction in *cis* proportion. Patent Document 4 discloses a process for preparing 1,4-cyclohexanedicarboxylic dichloride having more than 60% of the cis isomer via reaction of a mixture of the corresponding diacid comprising predominantly the cis-isomer with thionyl chloride at low temperature (50 °C). Removal of the acidic gas under reduced pressure and distillation of the residue using a thin-film distiller is also disclosed.

Patent Document 5 discloses a method of preparing pure 1,4-cyclohexanedicarboxylic dichloride (68:32 cis:trans) from the corresponding acid (80:20 cis:trans) via reaction with thionyl chloride in DMF at 60 °C, wherein the reaction mixture is then degassed (removal of acidic gases) and purified by distillation under reduced pressure.

Patent Document 6 discloses a method of preparing pure 1,4-cyclohexanedicarboxylic dichloride (6:4 cis:trans) from the corresponding acid (7:3 cis:trans) via reaction with thionyl chloride in the presence of DMF at reflux (i.e. ca 75°C for thionyl chloride), wherein the reaction mixture is then diluted in toluene and purified by distillation under reduced pressure.

Under such circumstances, there is a need for 1,4-cyclohexanedicarboxylic acid dichloride containing few yellow-coloring components and few acidic gas components, predominantly containing *cis* isomers, which are industrially easy to handle, and liquid at normal temperature, but such 1,4-cyclohexanedicarboxylic acid dichloride has been difficult to prepare.

Prior Art Documents

Patent Documents

Patent Document 1: JP 58-198439 A
Patent Document 2: JP 2009-256313 A
Patent Document 3: JP 2014-185119 A
Patent Document 4: JP 2015-172036 A
Patent Document 5: JP 2014-047201 A
Patent Document 6: JP 2014-196288 A

Summary of the Invention

Problems to be Solved by the Invention

There has been a need to develop methods for preparing 1,4-cyclohexanedicarboxylic acid dichloride that is colorless and transparent, contains few acidic gas components, and predominantly contains *cis* isomers, which are industrially easy to handle.

Means for Solving the Problems

The inventor intensively studied to solve the problems described above to discover a method for preparing 1,4-cyclohexanedicarboxylic acid dichloride containing few yellow-coloring components and having a small reduction in *cis* proportion, thereby completing the present invention.
Thus, the present invention is a method for preparing 1,4-cyclohexanedicarboxylic acid dichloride including reacting 1,4-cyclohexanedicarboxylic acid containing at least 70% *cis* isomers with thionyl chloride, wherein the 1,4-cyclohexanedicarboxylic acid dichloride contains at least 60% *cis* isomers, and the 1,4-cyclohexanedicarboxylic acid dichloride contains acidic gas in an amount of 1.0% by weight or less,
wherein the reaction is carried out at 40 to 50 °C by adding thionyl chloride dropwise to 1,4-cyclohexanecarboxylic acid, further comprising removing unreacted thionyl chloride and generated acidic gas after the reaction,
wherein the removal of unreacted thionyl chloride and generated acidic gas is carried out by degassing, and the degassing is carried out by passing nitrogen through the reaction solution at a reduced pressure of 13.3 kPa or lower and at a temperature of 80°C or lower.

Effects of the Invention

The 1,4-cyclohexanedicarboxylic acid dichloride prepared by the present invention contains predominantly *cis* isomers and few acidic gas components. The 1,4-cyclohexanedicarboxylic acid dichloride can be used as a raw material of various resins. Synthetic resins, resin additives, and pharmaceutical intermediates produced using the 1,4-cyclohexanedicarboxylic acid dichloride prepared by the present invention are accompanied by few by-products derived from acidic gas.

Mode for Carrying Out the Invention

The present invention is a method for preparing 1,4-cyclohexanedicarboxylic acid dichloride including reacting 1,4-cyclohexanedicarboxylic acid containing at least 70% *cis* isomers with thionyl chloride, wherein the 1,4-cyclohexanedicarboxylic acid dichloride contains at least 60% *cis* isomers, and the 1,4-cyclohexanedicarboxylic acid dichloride contains acidic gas in an amount of 1.0% by weight or less, wherein the reaction is carried out at 40 to 50 °C by adding thionyl chloride dropwise to 1,4-cyclohexanecarboxylic acid, further comprising removing unreacted thionyl chloride and generated acidic gas after the reaction, wherein the removal of unreacted thionyl chloride and generated acidic gas is carried out by degassing, and the degassing is carried out by passing nitrogen through the reaction solution at a reduced pressure of 13.3 kPa or lower and at a temperature of 80°C or lower.

The present invention will be described in detail.

In the method for preparing 1,4-cyclohexanedicarboxylic acid dichloride according to the present invention, 1,4-cyclohexanedicarboxylic acid and thionyl chloride are reacted. The amount of thionyl chloride used in the reaction is preferably 2.0 to 3.0 equivalents, more preferably 2.0 to 2.5 equivalents, relative to the amount of carboxylic acid used as a reactant. The reaction temperature in this process is 40 to 50 °C because isomerization from *cis* to *trans* occurs at high temperatures and because such isomerization can be further inhibited.

Typically, 1,4-cyclohexanedicarboxylic acid, used as a reactant, is prepared by nuclear hydrogenation of terephthalic acid. In general, 1,4-cyclohexanedicarboxylic acid predominantly contains *cis* isomers. The 1,4-cyclohexanedicarboxylic acid for use as a reactant in the present invention is 1,4-cyclohexanedicarboxylic acid containing at least 70% *cis* isomers.

Upon reaction or heating, 1,4-cyclohexanedicarboxylic acid isomerizes from *cis* to *trans.* The isomerization increases the proportion of *trans* isomers, and the resulting 1,4-cyclohexanedicarboxylic acid dichloride may be solidified at normal temperature. The solidified 1,4-cyclohexanedicarboxylic acid dichloride, when used in a subsequent reaction, must be dissolved once, which may lead to complexity in industrial handling and increased cost. Thus, the reduction in *cis* proportion from 1,4-cyclohexanedicarboxylic acid, used as a reactant, to 1,4-cyclohexanedicarboxylic acid dichloride, obtained as a product, is preferably 20% or lower, more preferably 10% or lower.

Typically, 1,4-cyclohexanedicarboxylic acid dichloride, which is an acid chloride and has high reactivity, is converted into synthetic resins and additives. However, unreacted thionyl chloride and acidic gas, if present, may inhibit the reaction and cause side reactions to generate impurities during the process of converting into a synthetic resin or an additive, leading to a low yield. In the method for preparing 1,4-cyclohexanedicarboxylic acid dichloride according to the present invention, the amount of acidic gas in 1,4-cyclohexanedicarboxylic acid dichloride is 1.0% by weight or less, preferably 0.5% by weight or less.

In the present invention, the term "acidic gas" refers to a gas having acidity. Typically, the acidic gas that will be generated when 1,4-cyclohexanedicarboxylic acid and thionyl chloride are reacted is preferably hydrogen chloride and sulfur dioxide. In the method for preparing 1,4-cyclohexanedicarboxylic acid dichloride according to the present invention, the amount of sulfur dioxide in 1,4-cyclohexanedicarboxylic acid dichloride is preferably 1.0% by weight or less. In the method for preparing 1,4-cyclohexanedicarboxylic acid dichloride according to the present invention, the amount of sulfur dioxide in 1,4-cyclohexanedicarboxylic acid dichloride is more preferably 0.5% by weight or less.

Preferably, in the present invention, the reaction is carried out at 40 to 50 °C or lower by adding thionyl chloride dropwise to 1,4-cyclohexanedicarboxylic acid, and then unreacted thionyl chloride and generated acidic gas are removed.

The step of removing unreacted thionyl chloride and generated acidic gas is preferably carried out under reduced pressure. The reduced pressure is 13.3 kPa or lower, more preferably 6.67 kPa or lower. The degassing temperature is 80°C or lower, more preferably 40°C to 50°C.

To remove unreacted thionyl chloride and acidic gas in 1,4-cyclohexanedicarboxylic acid dichloride, nitrogen is passed through the reaction solution. For example, when a 2000 L reaction can is used, the rate of passing the gas is preferably 1 L/Hr or more, particularly preferably 3 L/Hr or more. Furthermore, unreacted thionyl chloride and generated acidic gas are removed under reduced pressure. When unreacted thionyl chloride and generated acidic gas are removed, the reaction solution bubbles furiously, and thus the passing gas is preferably passed after the bubbling has subsided.

In particular, the reduced pressure during the step of removing unreacted thionyl chloride and generated acidic gas is 13.3 kPa or lower, preferably 6.67 kPa or lower. The temperature during the step is 80°C or lower, preferably 40°C to 50°C.

In the step of removing unreacted thionyl chloride and generated acidic gas, the pressure is preferably reduced gradually from normal pressure while observing the state of the reaction solution to control bubbling. If the pressure is reduced rapidly, a large excess of acidic gas components, which exist in the reaction solution at the start of degassing, are removed to cause bubbling. The passing is preferably carried out by introduction through a bottom stop valve of a reaction can or introduction through an internal tube into the reaction solution.

The 1,4-cyclohexanedicarboxylic acid dichloride thus obtained containing acidic gas components in an amount of 1.0% by weight or less is, in most cases, a transparent or opaque solution colored yellow. Presumably, this coloring is due to yellow coloring components in thionyl chloride and gradually proceeds during the reaction of 1,4-cyclohexanedicarboxylic acid and thionyl chloride. When the colored 1,4-cyclohexanedicarboxylic acid dichloride is used as it is, the coloring components may be incorporated into synthetic resins and resin additives, which cannot be used as products. Thus, the colored 1,4-cyclohexanedicarboxylic acid dichloride is preferably distilled to be colorless and transparent.

If the colored 1,4-cyclohexanedicarboxylic acid dichloride is distilled by a usual method, isomerization from *cis* to *trans* proceeds gradually due to thermal hysteresis to a *cis* proportion of 50% or less. Thus, it is preferable to distill 1,4-cyclohexanedicarboxylic acid dichloride prepared by reacting 1,4-cyclohexanedicarboxylic acid containing at least 70% *cis* isomers with thionyl chloride using thin-film distiller. Since 1,4-cyclohexanedicarboxylic acid dichloride, when decomposed, generates hydrogen chloride, a stainless steel thin-film distiller would be rusted. Thus, a glass thin-film distiller is preferred. The 1,4-cyclohexanedicarboxylic acid dichloride thus obtained is colorless and transparent.

In the present invention, 1,4-cyclohexanedicarboxylic acid dichloride predominantly containing *cis* isomers that is colorless and liquid at normal temperature can be prepared, preferably, by thin-film distillation.

In the present invention, 1,4-cyclohexanedicarboxylic acid dichloride containing at least 60% *cis* isomers can be prepared. In the present invention, 1,4-cyclohexanedicarboxylic acid dichloride contains at least 60% *cis* isomers because such 1,4-cyclohexanedicarboxylic acid dichloride does not solidify at low temperatures.

The 1,4-cyclohexanedicarboxylic acid dichloride thus obtained contains few unwanted acidic gas components, which may be produced as by-products of the reaction, and causes few side reactions when used as a reactant in a subsequent reaction. Thus, the 1,4-cyclohexanedicarboxylic acid dichloride is very suitable as a raw material of synthetic resins, resin additives, and pharmaceutical intermediates. Examples

The present invention will now be described in more detail with reference to examples.

In the examples, the chemical purity and the *cis/trans* isomer ratio of 1,4-cyclohexanedicarboxylic acid dichloride were analyzed by gas chromatography (GC) as described below. Acidic components in 1,4-cyclohexanedicarboxylic acid dichloride were analyzed by the titration prescribed in JIS 1418-1958.

GC Analysis, Chemical Purity Analysis, *cis/trans* Analysis

Samples were esterified with isopropanol before analysis. The GC analysis was performed under the following conditions.

Column: InertCap1 (0.25 mm $\times$ 60 m $\times$ 0.4 $\mu$m)
Carrier gas: helium
Inlet temperature: 200°C
Detector temperature: 310°C
Column temperature: 100°C (0 min) $\rightarrow$ 10°C/min $\rightarrow$ 300°C (10 min)
Column flow rate: 3.65 mL/min
Purge flow rate: 5.0 mL/min
Split ratio: 20
Sample quantity: 1 $\mu$L
Retention time: *cis,* 13.1 min; *trans,* 13.5 min

Acidic Component Analysis

Unreacted thionyl chloride and acidic gas (sulfur dioxide) components were analyzed by the redox titration described in JIS 1418-1958. The analysis method will be described below.

1. One gram of 1,4-cyclohexanedicarboxylic acid dichloride was placed in a 200 mL stoppered Erlenmeyer flask and precisely weighed using a precision balance to four places of decimals.
2. Fifty milliliters of a IN aqueous sodium hydroxide solution was weighed using a 50 mL graduated cylinder and placed in the Erlenmeyer flask. The flask was purged with nitrogen, and the 1,4-cyclohexanedicarboxylic acid dichloride was dissolved.
3. About three drops of phenolphthalein were added to give a neutralized 20% aqueous sulfuric acid solution.
4. Fifty milliliters of a 0.1N iodine solution was added using a whole pipette, and then 2 mL of concentrated hydrochloric acid was added to acidify the mixed solution.
5. Excess iodine was back titrated with 0.1N sodium thiosulfate.
6. The titer of a blank was determined by carrying out the steps 2 to 5.
7. The $SO_2$ concentration was calculated using the following equation:

$$SO_2 \text{ content } (\%) = 64.07/2 \times 0.1 \text{ (N)} \times f \times (Tb - Ts)/M \times 100$$

(f: sodium thiosulfate factor, Tb: titer in blank analysis (mL), Ts: titer in sample analysis (mL), M: sample weight (mg)).

Reactants

1,4-Cyclohexanedicarboxylic acid: an industrial product available from Eastman (*cis/trans* ratio: 73.4/26.6)
Thionyl chloride: an industrial product available from Sumitomo Seika Chemicals Co., Ltd.

The isopropanol used for the analysis was a dehydrated isopropanol available from Kanto Kagaku.

Example 1

Method of preparing 1,4-cyclohexanedicarboxylic acid dichloride that contains at least 60% *cis* isomers, is colorless transparent liquid at normal temperature, has $SO_2$ content of 0.5% or less, and has reduction in *cis* proportion, as compared with reactant, of 5% or less
Into a 2000 L reaction can equipped with a thermometer, a stirrer, and a condenser, 895 kg (5.20 kmol) of 1,4-cyclohexanedicarboxylic acid was loaded, and then 1,361 kg (11.4 kmol) of thionyl chloride was loaded. The resulting mixture was stirred at 45°C to 50°C for about 8 hours to give a cloudy pale yellow solution. Thereafter, degassing was carried out in such a manner that the pressure is gradually reduced to 40 Torr at a controlled temperature of 45°C to 50°C while observing the state of bubbling in the can. After the bubbling in the can subsided, the degassing was continued in such a manner that nitrogen was passed through the reaction solution for 144 hours by introducing nitrogen through a bottom stop valve while maintaining the pressure at 47 Torr. The nitrogen flow rate was 10 L/Hr. The reaction solution was then cooled and distilled at 98°C to 100°C using a GL thin-film distiller while maintaining the pressure at 1.5 to 1.8 Torr to give 1,009 kg (4.83 kmol) of colorless transparent 1,4-cyclohexanedicarboxylic acid dichloride.
GC analysis showed that the chemical purity was 99.4%; the *cis/trans* ratio was 72.3/27.7; the *cis* reduction was 1.1; and the $SO_2$ content was 0.02%.

Example 2 (Reference)[1]

Method of preparing 1,4-cyclohexanedicarboxylic acid dichloride that contains at least 50% *cis* isomers and is colorless transparent liquid at 20°C to higher
[1]The reference examples are outside the scope of the invention
Under the conditions described in Example 1, 1,4-cyclohexanedicarboxylic acid and thionyl chloride were loaded. The resulting mixture was stirred at 68°C to 70°C for about 8 hours to give a cloudy pale yellow solution. Thereafter, the same procedure as in Example 1 was repeated at 68°C to 70°C, and nitrogen was passed for 66 hours. The nitrogen flow rate was 2 L/Hr. Thereafter, thin-film distillation was carried out in the same manner as in Example 1 to give 998 kg (4.77 kmol) of colorless transparent 1,4-cyclohexanedicarboxylic acid dichloride.
GC analysis showed that the chemical purity was 99.2%; the *cis/trans* ratio was 54.0/46.0; the *cis* reduction was 19.4; and the $SO_2$ content was 0.02%.

Example 3

Method of preparing 1,4-cyclohexanedicarboxylic acid dichloride that is colorless transparent liquid at normal temperature, has $SO_2$ content of 1.0% or less, and has reduction in *cis* proportion, as compared with reactant, of 5% or less
Into a 2000 L reaction can equipped with a thermometer, a stirrer, and a condenser, 1,4-cyclohexanedicarboxylic acid and thionyl chloride were loaded under the conditions described in Example 1. The resulting mixture was stirred at 45°C to 50°C for about 8 hours to give a cloudy yellow solution. Thereafter, degassing was carried out under the conditions described in Example 1 at a nitrogen flow rate of 2 L/Hr. Thereafter, thin-film distillation was carried out under the conditions described in Example 1 to give 995 kg (4.76 kmol) of colorless transparent 1,4-cyclohexanedicarboxylic acid dichloride.
GC analysis showed that the chemical purity was 99.3%; the *cis/trans* ratio was 72.8/27.2; the *cis* reduction was 0.6; and the $SO_2$ content was 0.95%.

Example 4

Method of preparing 1,4-cyclohexanedicarboxylic acid dichloride that contains at least 60% *cis* isomers, is opaque yellow solution at normal temperature, and has $SO_2$ content of 0.5% or less.
Under the conditions described in Example 1, 1,4-cyclohexanedicarboxylic acid and thionyl chloride were loaded, and the resulting mixture was stirred at 45°C to 50°C for about 8 hours to give a cloudy yellow solution. Thereafter, degassing was carried out under the conditions described in Example 1 to give 1,005 kg (4.81 kmol) of opaque yellow 1,4-cyclohexanedicarboxylic acid dichloride.
GC analysis showed that the chemical purity was 99.4%; the *cis/trans* ratio was 72.0/28.0; the *cis* reduction was 1.4; the $SO_2$ content was 0.02%; and the resulting product was colored yellow.

Comparative Example 1

Method of preparing 1,4-cyclohexanedicarboxylic acid dichloride that contains at least 60% *cis* isomers, is colorless transparent liquid at normal temperature, has $SO_2$ content of 1.0% or more, and has reduction in *cis* proportion, as compared with reactant, of 5% or less

Under the conditions described in Example 1, 1,4-cyclohexanedicarboxylic acid and thionyl chloride were loaded, and the resulting mixture was stirred at 45°C to 50°C for about 8 hours to give a cloudy yellow solution. Thereafter, degassing was carried out under the conditions described in Example 1, and the after bubbling in the can subsided, the degassing was continued for 144 hours. Thereafter, thin-film distillation was carried out under the conditions described in Example 1 to give 995 kg (4.76 kmol) of colorless transparent 1,4-cyclohexanedicarboxylic acid dichloride. In Comparative Example 1, the degassing was not continued in such a manner that nitrogen was passed through the reaction solution by introducing nitrogen through a bottom stop valve.

GC analysis showed that the chemical purity was 99.4%; the *cis/trans* ratio was 72.9/27.1; the *cis* reduction was 0.5; and the $SO_2$ content was 1.8%. Although the resulting product was returned to the reaction can and degassing was carried out at 45°C to 50°C and 40 Torr for 144 hours, the $SO_2$ content was 1.7%, with only a slight decrease.

Comparative Example 2

Method of preparing 1,4-cyclohexanedicarboxylic acid dichloride that contains at least 40% *cis* isomers, is white solid at normal temperature, and has $SO_2$ content of 0.5% or less

Under the conditions described in Example 1, 1,4-cyclohexanedicarboxylic acid and thionyl chloride were loaded, and the resulting mixture was stirred at 95°C to 100°C for about 8 hours to give a cloudy yellow solution. Thereafter, degassing was carried out at 95°C to 100°C, and the after bubbling in the can subsided, degassing was carried out at 95°C to 100°C for 60 hours. Thereafter, thin-film distillation was carried out under the conditions described in Example 1 to give 995 kg (4.76 kmol) of slightly colored transparent 1,4-cyclohexanedicarboxylic acid dichloride. However, the 1,4-cyclohexanedicarboxylic acid dichloride, after being left to stand at room temperature for a while, crystallized to become a white crystal.

GC analysis showed that the chemical purity was 99.4%; the *cis/trans* ratio was 42.5/57.5; the *cis* reduction was 30.9; and the $SO_2$ content was 0.02%.

**Claims**

1. A method for preparing 1,4-cyclohexanedicarboxylic acid dichloride, comprising reacting 1,4-cyclohexanedicarboxylic acid containing at least 70% *cis* isomers with thionyl chloride, wherein the 1,4-cyclohexanedicarboxylic acid dichloride contains at least 60% *cis* isomers, and the 1,4-cyclohexanedicarboxylic acid dichloride contains acidic gas in an amount of 1.0% by weight or less,
   wherein the reaction is carried out at 40 to 50 °C by adding thionyl chloride dropwise to 1,4-cyclohexanecarboxylic acid,
   further comprising removing unreacted thionyl chloride and generated acidic gas after the reaction,
   wherein the removal of unreacted thionyl chloride and generated acidic gas is carried out by degassing, and the degassing is carried out by passing nitrogen through the reaction solution at a reduced pressure of 13.3 kPa or lower and at a temperature of 80°C or lower.

2. The method for preparing 1,4-cyclohexanedicarboxylic acid dichloride according to claim 1, wherein the acidic gas is hydrogen chloride and sulfur dioxide.

3. The method for preparing 1,4-cyclohexanedicarboxylic acid dichloride according to any one of claims 1 to 2, wherein the acidic gas is sulfur dioxide, and the amount of sulfur dioxide in 1,4-cyclohexanedicarboxylic acid dichloride is 1.0% by weight or less.

4. The method for preparing 1,4-cyclohexanedicarboxylic acid dichloride according to any one of claims 1 to 3, further comprising distilling the 1,4-cyclohexanedicarboxylic acid dichloride, prepared by the method of claim 1, using a thin-film distiller.

**Patentansprüche**

1. Verfahren zur Herstellung von 1,4-Cyclohexandicarbonsäuredichlorid, umfassend das Umsetzen von 1,4-Cyclohexandicarbonsäure, enthaltend mindestens 70% cis-Isomeren, mit Thionylchlorid, worin das 1,4-Cyclohexandicarbonsäuredichlorid mindestens 60% cis-Isomere enthält und das 1,4-Cyclohexandicarbonsäuredichlorid saures Gas in einer Menge von 1,0% oder weniger enthält,

die Reaktion bei 40 bis 50 °C durchgeführt wird, indem Thionylchlorid tropfenweise zu 1,4-Cyclohexancarbonsäure hinzugefügt wird,

ferner umfassend das Entfernen von unreagiertem Thionylchlorid und erzeugtem saurem Gas nach der Reaktion, wobei die Entfernung von unreagiertem Thionylchlorid und erzeugtem saurem Gas durch Entgasung durchgeführt wird, und die Entgasung durchgeführt wird, indem Stickstoff durch die Reaktionslösung bei einem reduzierten Druck von 13,3 kPa oder niedriger und bei einer Temperatur von 80°C oder niedriger geleitet wird.

2.  Verfahren zur Herstellung von 1,4-Cyclohexandicarbonsäuredichlorid nach Anspruch 1, worin das saure Gas Chlorwasserstoff und Schwefeldioxid ist.

3.  Verfahren zur Herstellung von 1,4-Cyclohexandicarbonsäuredichlorid nach einem der Ansprüche 1 bis 2, worin das saure Gas Schwefeldioxid ist und die Menge an Schwefeldioxid in 1,4-Cyclohexandicarbonsäuredichlorid 1,0 Gew.-% oder weniger beträgt.

4.  Verfahren zur Herstellung von 1,4-Cyclohexandicarbonsäuredichlorid nach einem der Ansprüche 1 bis 3, ferner umfassend das Destillieren des 1,4-Cyclohexandicarbonsäuredichlorids, hergestellt nach dem Verfahren laut Anspruch 1, unter Verwendung eines Dünnschichtdestillierers.

## Revendications

1.  Procédé de préparation de dichlorure d'acide 1,4-cyclohexanedicarboxylique, comprenant la réaction d'acide 1,4-cyclohexanedicarboxylique contenant au moins 70% d'isomères cis avec du chlorure de thionyle, dans lequel le dichlorure d'acide 1,4-cyclohexanedicarboxylique contient au moins 60% d'isomères cis et le dichlorure d'acide 1,4-cyclohexanedicarboxylique contient un gaz acide en quantité inférieure à 1,0% en poids,
    dans laquelle la réaction est effectuée à une température de 40 à 50 °C par addition goutte à goutte de chlorure de thionyle à l'acide 1,4-cyclohexanecarboxylique,
    comprenant en outre l'élimination du chlorure de thionyle n'ayant pas réagi et du gaz acide généré après la réaction, dans laquelle l'élimination du chlorure de thionyle n'ayant pas réagi et du gaz acide généré est effectuée par dégazage, et le dégazage est effectué en faisant passer de l'azote à travers la solution réactionnelle à une pression réduite de 13,3 kPa ou moins et à une température égale ou inférieure à 80°C.

2.  Procédé de préparation de dichlorure d'acide 1,4-cyclohexanedicarboxylique selon la revendication 1, dans lequel le gaz acide est le chlorure d'hydrogène et le dioxyde de soufre.

3.  Procédé de préparation de dichlorure d'acide 1,4-cyclohexanedicarboxylique selon l'une quelconque des revendications 1 à 2, dans lequel le gaz acide est du dioxyde de soufre, et la quantité de dioxyde de soufre dans le dichlorure d'acide 1,4-cyclohexanedicarboxylique est de 1,0% en poids ou moins.

4.  Procédé de préparation de dichlorure d'acide 1,4-cyclohexanedicarboxylique selon l'une quelconque des revendications 1 à 3, comprenant en outre la distillation du dichlorure d'acide 1,4-cyclohexanedicarboxylique, préparé par le procédé selon la revendication 1, en utilisant un distillateur à film mince.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 58198439 A
- JP 2009256313 A
- JP 2014185119 A
- JP 2015172036 A
- JP 2014047201 A
- JP 2014196288 A